Europäisches Patentamt

European Patent Office

Office européen des brevets

(19)

(11) Numéro de publication: **0 108 017**
**B1**

## FASCICULE DE BREVET EUROPEEN

(12)

(45) Date de publication du fascicule du brevet:
**10.12.86**

(21) Numéro de dépôt: **83402084.4**

(22) Date de dépôt: **26.10.83**

(51) Int. Cl.⁴: **A 61 K 31/68**, A 61 K 45/06 //
(A61K31/68,
31:485),(A61K31/68,
31:535),(A61K31/68, 31:445)

(54) Association renfermant à titre de principes actifs, un analgésique central et la vitamine B12 ou un de ses analogues.

(30) Priorité: **26.10.82 FR 8217892**

(43) Date de publication de la demande:
**09.05.84 Bulletin 84/19**

(45) Mention de la délivrance du brevet:
**10.12.86 Bulletin 86/50**

(84) Etats contractants désignés:
**AT BE CH DE FR GB IT LI LU NL SE**

(56) Documents cités:
**FR - M - 3 039**

**UNLISTED DRUGS, vol. 27, no. 8, août 1975, page 130,
no. e, Chatham, New Jersey, US
ARZNEIMITTEL-FORSCHUNG, vol. 18, no. 5, mai 1968,
pages 625-628, Aulendorf/Württ., DE;E. SZIRMAI et al.:
"Experimentelle Untersuchungen mit einem neuen
Strahlenschutzpräparat"
UNLISTED DRUGS, vol. 21, no. 12, décembre 1969, page
1830, Chatham, New Jersey, US "Anarthral"
ROTE LISTE, 1971, page 352, Editio Cantor,
Aulendorf/Württ., DE "Dexa-Attritin 3000"**

(73) Titulaire: **ROUSSEL-UCLAF, 35, boulevard des Invalides,
F-75007 Paris (FR)**

(72) Inventeur: **Delevallee, Francoise, 55, rue Diderot,
F-94320 Vincennes (FR)**
Inventeur: **Deraedt, Roger, 23, Allée Jean-Baptiste
Clément, F-93320 Pavillons Sous Bois (FR)**

(74) Mandataire: **Fritel, Hubert et al, Département des
Brevets ROUSSEL UCLAF B.P. no 9,
F-93230 Romainville (FR)**

## Description

La présente invention a pour objet l'association renfermant à titre de principes actifs:

a) la morphine, la péthidine, la dextromoramide ou la pentazocine et

b) la vitamine B12 ou l'un de ses analogues, pour une utilisation simultanée ou séparée dans le temps, dans une méthode de traitement analgésique du corps humain ou animal.

Des associations antalgiques à base soit d'acide acétyl salicylique et d'hydroxocobalamine, soit de salicylamide et de cyanocobalamine ont déjà été décrits notamment dans le brevet Spécial de Médicament n° 3039 ou dans Unlisted Drugs, volume 21, n° 12, décembre 1969, page 183.

Cependant jamais encore n'avait été réalisée l'association d'un analgésique central de type morphinique avec la vitamine $B_{12}$ ou de l'un de ces analogues.

Les analgésiques centraux narcotiques sont considérés classiquement comme possédant les mêmes caractéristiques que la morphine, ils provoquent une toxicomanie et entraînent une dépression respiratoire.

Les dérivés de synthèse de la morphine les plus utilisés en thérapeutique sont la péthidine, le dextromoramide et la pentazocine. Les dérivés présentent les mêmes inconvénients que la morphine et la recherche de dérivés possédant le même puissant effet analgésique que la morphine et dépourvus d'action toxicomanogène est restée jusqu'à présent infructueuse.

Les analogues de la vitamine B12 ou cyanocobalamine utilisés dans l'association selon l'invention, sont de préférence l'hydroxocobalamine, la nitrocobalamine, la méthylcobalamine, le coenzyme de la vitamine B12 ou dibencozide, forme physiologique de la vitamine B12 directement active sans transformation.

On sait que la vitamine B12 et ses analogues jouent un rôle très important dans l'organisme humain au cours de divers processus biochimiques nécessitant la présence de cette vitamine.

La vitamine B12 est ses analogues sont ainsi utilisés, par exemple, dans le traitement des asthénies, des anémies, de l'amaigrissement.

Par ailleurs, la vitamine B12 et ses analogues sont doués de propriétés antalgiques, lorsqu'ils sont administrés à fortes doses chez l'homme.

Mais l'association de la vitamine B12 ou de l'un des ses analogues avec un analgésique central n'avait jamais été réalisée jusqu'à ce jour.

Il vient d'être trouvé que si l'on associe la vitamine B12 ou l'un de ses analogues avec un analgésique central, on aboutit à un effet analgésique supérieur à celui auquel on aurait pu s'attendre avec l'analgésique seul.

C'est ainsi qu'au cours d'une expérimentation chez l'animal, la potentialisation de l'effet d'un analgésique a été obtenue en l'associant à une dose non analgésique d'un équivalent de la vitamine B12.

Celui-ci peut-être présent, dans l'association utilisée en thérapeutique humaine, à une dose analgésique ou non analgésique.

L'association, objet de l'invention, permet donc d'obtenir une action analgésique importante, tout en employant une quantité d'analgésique central inférieure à celle qui serait nécessaire pour obtenir la même activité et de diminuer ainsi les effets secondaires de l'analgésique utilisé.

On voit évidemment l'intérêt de l'association objet de l'invention, en thérapeutique humaine, puisqu'elle permet de réduire les effets indésirables des différents analgésiques centraux employés en thérapeutique.

L'analgésique central utilisé dans l'association est de préférence la morphine ou un dérivé de synthèse succédané de la morphine. Parmi ces derniers, on retient plus particulièrement les analgésiques utilisés en thérapeutique cités précédemment, tels la péthidine, le dextromoramide ou la pentazocine.

Les effets indésirables liés à l'administration d'analgésiques dérivés de la morphine à des doses usuelles sont connus:

1. nausées, vomissements
2. constipation
3. dépression respiratoire
4. dépendance physique et/ou psychique au cours de traitements prolongés
5. syndrome de manque à l'arrêt du traitement comportant: mydriase, contractions musculaires, céphalées, sudation, vomissements, diarrhée, tachycardie, polypnée, hyperthermine, hypertension ...

L'effet de synergie analgésique, obtenu grâce à l'association se révèle donc particulièrement utile pour amoindrir la dépendance physique et psychique ainsi que l'accoutumance se développant à la suite d'administrations répétées d'analgésiques de type morphinique.

On peut donc parler de l'association vitamine B12 ou analogue de celle-ci avec un analgésique morphinique comme d'un «économiseur de morphine».

L'invention concerne donc notamment l'association caractérisée en ce que l'analgésique central qu'elle renferme est la morphine.

L'invention a plus particulièrement pour objet, l'association caractérisée en ce que l'analogue de la vitamine B12 est l'hydroxocobalamine, la nitrocobalamine, la méthylcobalamine ou le dibencozide, et tout spécialement l'association caractérisée en ce qu'elle renferme le dibencozide.

La potentialisation de l'effet de l'analgésique central est illustrée par des études pharmacologiques chez le rat ou la souris, rapportées plus loin.

L'effet potentialisateur a ainsi été observé avec différents analgésiques, tels la morphine, un dérivé d'enképhaline substitué en 2, et le 5 hydroxytryptophane, précurseur de la sérotonine, celle-ci étant un médiateur de la réaction douloureuse.

D'après le résultat de ces études, l'association permet par exemple de réduire d'environ 2 à 4 fois la dose de morphine employée.

L'invention s'étend aux compositions pharmaceutiques renfermant l'association, objet de l'invention.

Les compositions pharmaceutiques peuvent être utilisées dans le traitement des douleurs intenses, en particulier rebelles aux antalgiques périphériques, par exemple, au cours des processus néoplasiques, dans le traitement des pancréatites, coliques néphrétiques ou biliaires, dans le traitement des douleurs post opératoires et post traumatiques.

Les compositions pharmaceutiques peuvent être administrées par voie buccale, parentérale ou rectale.

Ces compositions pharmaceutiques peuvent être, par exemple, solides ou liquides et se présenter sous les formes pharmaceutiques couramment utilisées en médecine humaine comme, par exemple, les comprimés, simples ou dragéifiés, les gélules, les capsules, les granulés, les préparations injectables, les suppositoires; elles sont préparées selon les méthodes usuelles. Le ou les principes actifs peuvent y être incorporés à des excipients habituellemtn employés dans ces compositions pharmaceutiques, tels que le talc, la gomme arabique, le lactose, l'amidon, le stéarate de magnésium, les véhicules auquex ou non, les corps gras d'origine animale ou végétale, les dérivés paraffiniques, les glycols, les divers agents mouillants, dispersants ou émulsifiants, les conservateurs.

Les proportions relatives des constituants de l'association, objet de l'invention, sont variables. Elles peuvent être, par exemple, de 1 partie environ d'analgésique pour environ 1 à 5 parties de vitamine B12 ou d'analogue de celle-ci.

La posologie varie en fonction de la voie d'administration, de l'affection traitée, du sujet en cause et de l'analgésique employé.

Les deux constituants de l'association selon l'invention peuvent être administrés en mélange, préparé extemporanément ou non. Ils peuvent aussi être administrés successivement, à des intervalles pouvant aller de quelques secondes à une ou deux heures, voire davantage (jusqu'à 15 heures).

Comme il a été dit précédemment, l'association permet de réduire de 2 à 4 fois la dose d'analgésique utilisé; ainsi la dose quotidienne de morphine chez l'adulte par voie parentérale habituellement de 0,005 g à 0,03 g peut être dans le cas de l'association morphine-dibencozide de 0,001 g à 0,01 g.

Les exemples suivants illustrent l'invention sans toutefois la limiter:

Exemple 1
Compositions pharmaceutiques

On a préparé extemporanément un soluté injectable répondant à la formule:
– soluté injectable de chlorhydrate
   de morphine 0,25%     2 ml
– dibencozide     20 mg

On prépare le soluté injectable en introduisant au moment de l'emploi le soluté injectable de chlorhydrate de morphine à 0,25% sur le dibencozide lyophilisé et en agitant.

On a préparé un deuxième soluté injectable répondant à la formule:
– chlorhydrate de morphine     5 mg
– cyanocobalamine     5 mg
– solvant stérile     2 ml

Exemple 2
Etude pharmacologique

a) Potentialisation de l'activité analgésique de la morphine sur le test de la plaque chaude chez le rat.

Des rats mâles, d'un poids moyen de 110 g, sont placés, un par un, sur une plaque de cuivre maintenue à 56 °C: la réaction à la douleur se manifeste par le léchage d'une patte ou le saut de l'animal; le temps de cette réaction est noté et l'on ne retient que les rats réagissant en moins de 10 secondes.

Les rats sont répartis en groupes homogènes, un groupe ne recevant que le véhicule des produits administrés.

Le dibencozide est administré à la dose inactive par elle-même de 10 mg/kg par voie sous-cutanée 30 minutes avant différentes doses de chlorhydrate de morphine également administrées par voie sous-cutanée. Dans les mêmes conditions de traitement, un groupe d'animaux ne reçoit que le dibencozide, un autre groupe ne reçoit que le chlorhydrate de morphine. La réactivité des rats à la douleur est notées 30, 60 et 90 minutes après le traitement.

Dans ces conditions, le dibencozide diminue la dose de morphine nécessaire pour doubler le temps de réaction des animaux à la douleur: 2,7 mg/Kg au lieu de 7,3 mg/Kg morphine.

b) Action du dibencozide sur le 5-hydroxytryptophane (5HTP) sur le test de la plaque chaude chez le rat.

Administré par voie sous-cutanée à la dose de 10 mg/Kg une heure après administration sous-cutanée d'une dose inactive de 5HTP, le dibencozide, qui n'a pratiquement pas d'effet propre, confère à ce dernier une activité analgésique.

Augmentation du temps de réaction en %:

| | 90 minutes | 120 minutes | 150 minutes |
|---|---|---|---|
| 5HTP (200 mg/kg) | 24 | 16 | 34 |
| Dibencozide | 0 | 7 | 23 |
| 5HTP | 82 | 71 | 84 |

c) Potentialisation de l'activité analgésique d'une enképhaline (E) sur le test de la plaque chaude chez la souris

Le dibencozide administré par voie sous-cutanée à la dose de 10 mg/Kg, 45 minutes avant l'injection intracérébroventriculaire d'une dose de 0,25 mg/Kg de D Ala$^2$-Met$^5$-enképhaline, augmente l'effet analgésique de celle-ci à 15 minutes.

Augmentation du temps de réaction en %:

| | 15 minutes |
|---|---|
| E | 29 |
| Dibencozide + E | 114 |

**Revendications pour les Etats contractants BE, CH, DE, FR, GB, IT, LI, LU, NL, SE**

1. Associaton renfermant, à titre de principes actifs:

a) la morphine, la péthidine, le dextromoramide ou la pentazocine et

b) la vitamine $B_{12}$ ou l'un de ses analogues, pour une utilisation simultanée ou séparée dans le temps, dans une méthode de traitement analgésique du corps humain ou animal.

2. Association selon la revendication 1, caractérisée en ce que l'analgésique central qu'elle renferme est la morphine.

3. Association selon la revendication 1 ou 2, caractérisée en ce que l'analogue de la vitaminc $B_{12}$ est le coenzyme de la vitamine $B_{12}$ ou dibencozide, l'hydroxocobalamine, la méthylcobalamine ou la nitrocobalamine.

4. Association selon la revendication 3, caractérisée en ce que l'analogue de la vitamine $B_{12}$ est le dibencozide.

5. Les compositions pharmaceutiques contenant à titre de principe actif une association renfermant:

a) la morophine, la péthidine, le dextromoramide, le pentazocine.

b) la vitamine $B_{12}$ ou l'un de ses analogues.

**Revendications pour l'Etat contractant AT**

1. Association renfermant, à titre de principes actifs:

a) la morphine, la péthidine, le dextromoramide ou la pentazocine et

b) la vitamine $B_{12}$ ou l'un de ses analogues, pour une utilisation simultanée ou séparée dans le temps, dans une méthode de traitement analgésique du corps humain ou animal.

2. Association selon la revendication 1, caractérisé en ce que l'analgésique central qu'elle renferme est la morphine.

3. Association selon la revendication 1 ou 2, caractérisée en ce que l'analogue de la vitamine $B_{12}$ est le coenzyme de la vitamine $B_{12}$ ou dibencozide, l'hydroxocobalamine, la méthylcobalamine ou la nitrocobalamine.

4. Association selon la revendication 3, caractérisée en ce que l'analogue de la vitamine $B_{12}$ est le dibencozide.

5. Les compositions pharmaceutiques contenant à titre de principe actif une association renfermant:

a) la morphine, la péthidine, le dextromoramide, la pentazocine.

b) la vitamine $B_{12}$ ou l'un de ses analogues.

6. Procédé de préparation des compositions pharmaceutiques telles que définies à la revendication 5, caractérisé en ce que l'on mélange ou juxtapose dans un même conditionnement pharmaceutique un analgésique central et la vitamine $B_{12}$ ou l'un de ses analogues.

**Patentansprüche für die Vertragsstaaten BE, CH, DE, FR, GB, IT, LI, LU, NL, SE**

1. Assoziation, enthaltend als Wirkstoff

(a) Morphin, Pethidin, Dextromoramid oder Pentazocin und

(b) Vitamin B12 oder eines seiner Analoga, für eine gleichzeitig oder zeitlich getrennte Anwendung bei einem Verfahren zur analgetischen Behandlung des menschlichen oder tierischen Körpers.

2. Assoziation gemäss Anspruch 1, dadurch gekennzeichnet, dass das zentrale Analgetikum, das sie enthält, Morphin ist.

3. Assoziation gemäss Ansprurch 1 oder 2, dadurch gekennzeichnet, dass das Analoge des Vitamins B12 das Co-Enzym des Vitamin B12 oder Dibencozid, Hydroxocobalamin, Methylcobalamin oder Nitrocobalamin ist.

4. Assoziation gemäss Anspruch 3, dadurch gekennzeichnet, dass das Analoge des Vitamin B12 Dibencozid ist.

5. Pharmazeutische Zusammensetzungen, enthaltend als Wirkstoff eine Assoziation, die

(a) Morphin, Pethidin, Dextromoramid oder Pentazocin und

(b) Vitamin B12 oder eines seiner Analoga umfasst.

**Patentansprüche für den Vertragsstaat AT**

1. Assoziation, enthaltend als Wirkstoffe

(a) Morphin, Pethidin, Dextromoramid oder Pentazocin und

(b) Vitamin B12 oder eines seiner Analoga, für die gleichzeitige oder zeitlich getrennte Verwendung bei einem Verfahren zur analgetischen Behandlung des menschlichen oder tierischen Körpers.

2. Assoziation gemäss Anspruch 1, dadurch gekennzeichnet, dass das zentrale Analgetikum, das sie enthält, Morphin ist.

3. Assoziation gemäss Anspruch 1 oder 2, dadurch gekennzeichnet, dass das Analoge des Vitamin B12 das Co-Enzym des Vitamin B12 oder Dibencozid, Hydroxocobalamin, Methylcobalamin oder Nitrocobalamin ist.

4. Assoziation gemäss Anspruch 3, dadurch gekennzeichnet, dass das Analoge des Vitamin B12 Dibencozid ist.

5. Pharmazeutische Zusammensetzungen, enthaltend als Wirkstoff eine Assoziation, umfassend

(a) Morphin, Pethidin, Dextromoramid oder Pentazocin, und

(b) Vitamin B12 oder eines seiner Analoga.

6. Verfahren zur Herstellung von pharmazeutischen Zusammensetzungen gemäss Anspruch 5, dadurch gekennzeichnet, dass man ein zentrales Analgetikum und Vitamin B12 oder eines seiner Analoga in einer gleichen pharmazeutischen Konditionierung mischt oder nebeneinanderstellt.

**Claims for the Contracting States BE, CH, DE, FR, GB, IT, LI, LU, NL, SE**

1. Combination containing as active principles:

a) morphine, pethidine, dextromoramide or pentazocine, and

b) vitamine $B_{12}$ or one of its analogues, for use simultaneously or separated in time, in an analgesic treatment method for the human or animal body.

2. Combination according to claim 1, characterized in that the central analgesic which it contains is morphine.

3. Combination according to claim 1 or 2, characterized in that the analogue of vitamine $B_{12}$ is the coenzyme of vitamine $B_{12}$ or dibencozide, hydroxocobalamine, methylcobalamine or nitrocobalamine.

4. Combination according to claim 3, characterized in that the analogue of vitamine $B_{12}$ is dibencozide.

5. Pharmaceutical compositions containing as active principle a combination containing:

a) morphine, pethidine, dextromoramide, pentazocine.

b) vitamine $B_{12}$ or one of its analogues.

**Claims for the Contracting State AT**

1. Combination containing as active principles:

a) morphine, pethidine, dextromoramide or pentazocine, and

b) vitamine $B_{12}$ or one of its analogues, for use simultaneously or separated in time, in an analgesic treatment method for the human or animal body.

2. Combination according to claim 1, characterized in that the central analgesic which it contains is morphine.

3. Combination according to claim 1 or 2, characterized in that the analogue of vitamine $B_{12}$ is the coenzyme of vitamine $B_{12}$ or dibencozide, hydroxocobalamine, methylcobalamine or nitrocobalamine.

4. Combination according to claim 3, characterized in that the analogue of vitamine $B_{12}$ is dibencozide.

5. Pharmaceutical compositions containing as active principle a combination containing:

a) morphine, pethidine, dextromoramide, pentazocine.

b) vitamine $B_{12}$ or one of its analogues.

6. Preparation process for pharmaceutical compositions as defined in claim 5, characterized in that there are mixed together in the same pharmaceutical packaging a central analgesic and the vitamine $B_{12}$ or one of its analogues.